Europäisches Patentamt

(19) European Patent Office

Office européen des brevets

(11) Publication number: **0 018 696**
**B1**

## EUROPEAN PATENT SPECIFICATION

(45) Date of publication of patent specification: **01.09.82**

(51) Int. Cl.³: **C 07 D  251/60**

(21) Application number: **80200405.1**

(22) Date of filing: **01.05.80**

(54) Method for the preparation of melamine.

(30) Priority: **03.05.79 NL 7903473**
**04.10.79 NL 7907368**

(43) Date of publication of application:
**12.11.80 Bulletin 80/23**

(45) Publication of the grant of the patent:
**01.09.82 Bulletin 82/35**

(84) Designated Contracting States:
**AT BE DE FR GB IT NL SE**

(56) References cited:
**GB - A - 1 237 780**

(73) Proprietor: **STAMICARBON B.V.**
**Postbus 10**
**NL-6160 MC  Geleen (NL)**

(72) Inventor: **van Hardeveld, Rudolf**
**Vondellaan 15**
**NL-6165 EB Geleen (NL)**

(74) Representative: **Hatzmann, Marinus Jan et al,**
**OCTROOIBUREAU DSM P.O.Box 9**
**NL-6160 MA Geleen (NL)**

Courier Press, Leamington Spa, England.

## Method for the preparation of melamine

The invention relates to a method for the preparation of melamine by converting urea and/or thermal decomposition product thereof in a fluid bed of catalytically active material in the presence of a gas mixture containing $NH_3$ and $CO_2$, using a gasmixture of ammonia and carbondioxide as the fluidizing gas.

From Hydrocarbon Processing, September 1969, pp. 184—186, it is known to prepare melamine at atmospheric pressure and to subsequently separate it from the reaction gases formed by means of a so-called 'dry catch'. The gases freed of melamine, consisting mainly of $NH_3$ and $CO_2$, are partly recirculated as fluidizing gas for the fluid bed of catalytically active material.

This method has the disadvantage that, at the pressure applied and the partial pressure of ammonia corresponding with it, so many deamination products of melamine are formed that it is necessary to remove these products prior to the separation of melamine from the reaction gases. This necessitates a hot gas filtration, which requires substantial investments and which is laborious.

In the British patent specification 1,237,780 a process for preparing melamine is described in which the off gas-mixture obtained after separating melamine therefrom is further cooled to remove carbondioxide as ammoniumcarbonate. In this process only ammonia is used as fluidizing gas.

The purpose of the invention is to provide a method for the preparation of melamine in which hardly any or no deamination products are formed.

According to the invention this method is characterized in that the partial pressure of ammonia in the fluid bed is at least 70 kPa.

The fact is that, surprisingly, it has been found that at partial pressures of ammonia of a minimum of 70 kPa already very few deamination products are formed.

In a preferred embodiment of the method according to the invention the partial pressure of $NH_3$ is at least 100 kPa, more specifically 350 kPa.

In the prior art processes for preparing melamine at atmospheric pressure, using a gas mixture of ammonia and carbondioxide as fluidizing gas, the ammonia partial pressure in the fluidized bed is about 50—60 kPa.

In the medium pressure processes ammonia partial pressure higher than 70 kPa may occur. In these processes, however, ammonia is used as fluidizing gas. The ratio of ammonia to carbondioxide (molar, or by volume, which is the same) in the fluidized bed is in these processes about 90—95 to 10—5.

The separation of melamine from the gases coming from the reactor can be effected by direct or indirect cooling. For instance, cold $NH_3$ gas or gas mixtures of $NH_3$ and $CO_2$ can be mixed with reaction gases.

Another possibility is the application of a desublimation of melamine in one or more fluid beds of melamine particles with application of indirect cooling. This indirect cooling will preferably be effected with cooling water, in which process, by running the cooling water counter current to the melamine reaction gases, high-quality steam is formed.

The advantage of this method is that relatively large melamine particles are obtained with a relatively narrow particle size distribution. Moreover, the melamine obtained in this manner is free flowing, so that it can be conveyed in bulk, which is not the case when direct cooling is applied.

A process of this kind is described in the copending application 80200403.6 publication number 18.695, of the same date.

The gas mixture of $NH_3$ and $CO_2$, which is used as fluidizing gas in the melamine reactor, preferably consists of 90% by weight of $NH_3$ and $CO_2$, while the volume ratio between $NH_3$ and $CO_2$ may be between 1.5—1 and 2.5—1.

The total pressure in the fluid bed is preferably 2,500 kPa at most, more specifically 1,250 kPa at most.

As catalyst in the fluid bed in the reactor one of the known catalysts can be used, such as aluminium oxide, aluminium oxide on silicon oxide, silicon oxide, titanium oxide, zirconium oxide, boron phosphate or aluminium phosphate or a mixture of 2 or more of these catalysts. The expression catalyst or catalytically active material is understood here to mean any material promoting, under the reaction conditions applied, the conversion of urea into melamine.

The temperature at which the melamine is formed from urea is generally more than 325°C. In general, this temperature will not be about 460°C, more specifically preference is given to temperatures of between 370 and 400°C. The temperature applied depends, in part, on the pressure in the reactor.

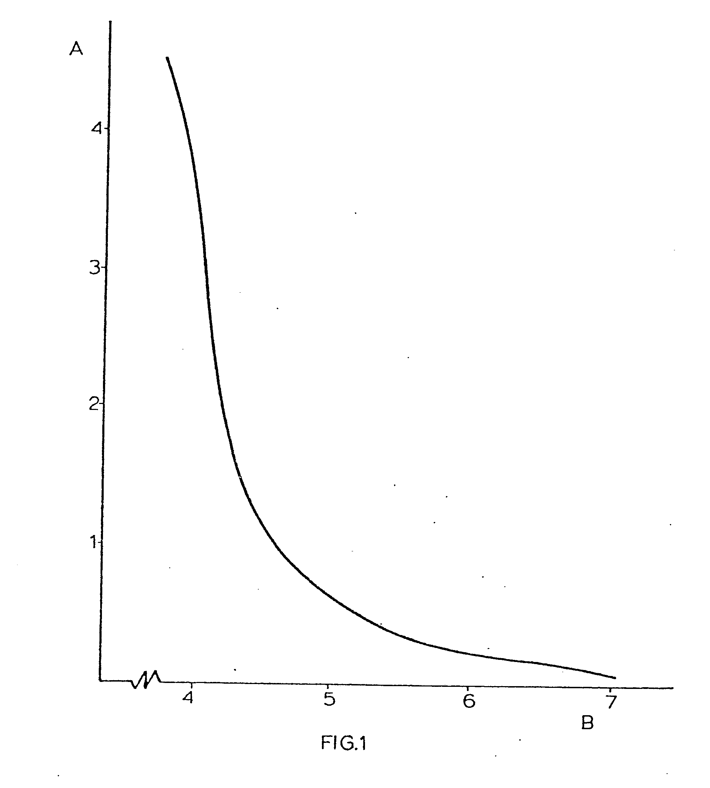

The invention will now be elucidated by means of a drawing in which fig. 1 gives a graph in which the content of deamination products in the reactor gases has been plotted as a function of the natural logarithm of the partial pressure of ammonia in the fluid bed and

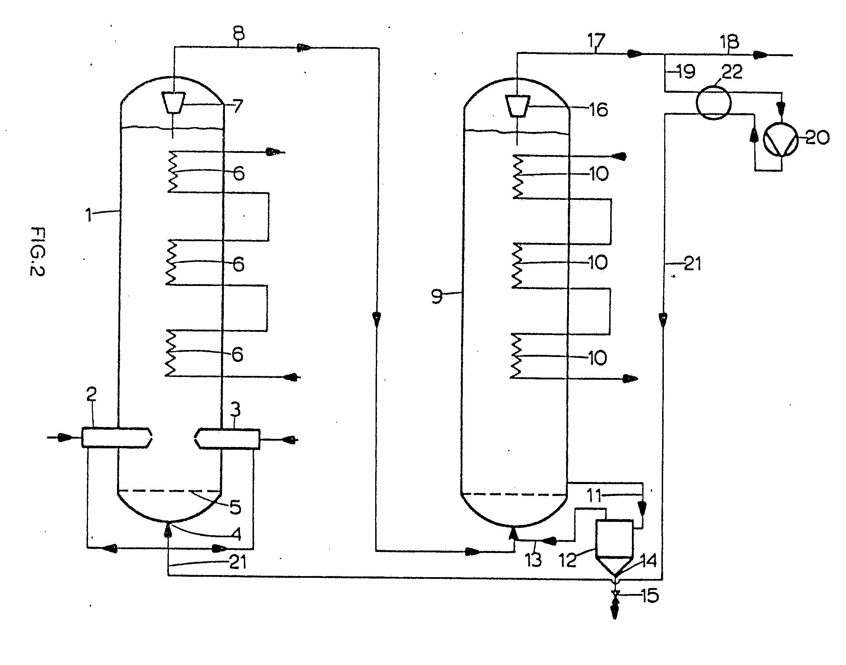

fig. 2 gives a diagrammatic embodiment of a method for the preparation of melamine in which the method according to the invention can be applied.

From fig. 1, in which the relative content of deamination products in the melamine-containing reaction gas (A) has been plotted as a function of the natural logarithm of the partial pressure of ammonia (B, kPa) in this gas, it is clear that as the partial pressure increases this content decreases very strongly. The content of deamination products has been given in respect of the value at the $NH_3$ partial pressure of 100 Kpa, which has been fixed at 1.

In figure 2 a diagrammatic embodiment of the method according to the invention has been given.

In reactor 1 urea is atomized, through sprayers 2, 3, in a fluid bed of catalyst particles. This bed is fluidized by means of a gas mixture supplied through fluidizing gas supply 4 and gas distributor plate 5. In the fluid bed the desired temperature is maintained by means of heat exchanger pipes 6, which have been shown here diagrammatically. The melamine-containing reactor gases are fed, through cyclone 7 and line 8, to desublimator 9. In this desublimator 9, which is in the form of one or more fluid beds of melamine particles, melamine is desublimated.

Through heat exchanger tubes 10, the heat released in this process is removed and converted into high pressure steam.

At the bottom of desublimator 9 solid melamine particles are removed through line 11. In separator 12 melamine is separated from the gases. The gases are returned, through line 13, to the desublimator 9. Through line 14, and after pressure reduction via valve 15, the melamine is removed.

Through cyclone 16 and line 17 the off gas mixture, freed of melamine, is removed. A part of the off gas mixture is removed through line 18, for instance to a urea synthesis, an $NH_3$—$CO_2$ separation or a fertilizer preparation. The remaining part is recirculated, through line 19, compressor 20, line 21 and heat exchanger 22, to the reactor 1.

The invention will now be elucidated by means of an example but is not restricted to it.

### Example

The melamine preparation was performed in an installation described in figure 2.

To the melamine reactor, operating at a pressure of 1,000 kPa and a temperature of 375°C, 20,000 kg of urea and 30,000 kg of gaseous $NH_3$ and $CO_2$ were fed per hour. The partial pressure of $NH_3$ was 600 kPa.

The reaction gases, containing melamine vapours, were fed to desublimator 9, which also operated at a pressure of 900 kPa. This desublimator contained a fluid bed with melamine particles. Per hour about 7,000 kg of melamine with good free-flowing properties and a purity of 99.9% was discharged from the desublimator.

Of the off gas mixture, largely freed of melamine, 80% was recirculated to the melamine reactor, after compression to compensate for the fall in pressure across the reactor and the desublimator, and heat exchange. The remaining 20% was fed to an $NH_3/CH_2$ separation.

## Claims

1. Method for the preparation of melamine by converting urea and/or thermal decomposition products thereof in a fluid bed of catalytically active material in the presence of a gas mixture containing $NH_3$ and $CO_2$, using a gasmixture of $NH_3$ and $CO_2$ as the fluidizing gas, characterized in that the partial pressure of ammonia in the fluid bed is at least 70 kPa.

2. Method according to claim 1, characterized in that the partial pressure of $NH_3$ is at least 100 kPa.

3. Method according to claim 2, characterized in that the partial pressure of $NH_3$ is at least 350 kPa.

4. Method according to claim 1, characterized in that the gas mixture consists for at least 90% of $NH_3$ and $CO_2$.

5. Method according to claim 1, characterized in that the volume ratio of $NH_3$ to $CO_2$ is between 1.5—1 and 2.5—1.

6. Method according to claim 1, characterized in that the total pressure in the fluid bed is 2,500 kPa at most.

7. Method according to claim 1, characterized in that the total pressure in the fluid bed is 1,250 kPa at most.

## Patentansprüche

1. Verfahren zur Herstellung von Melamin, indem man Harnstoff und/oder thermische Zersetzungsprodukte dieses Stoffes in einem aus katalytisch aktiver Masse bestehenden Fleissbett in Anwesenheit eines $NH_3$ und $CO_2$ enthaltenden Gasgemisches umsetzt, unter Verwendung eines Gasgemisches aus $NH_3$ und $CO_2$ als Fliessgas, dadurch gekennzeichnet, dass der Partialdruck von Ammoniak im Fliessbett zumindest 70 kPa beträgt.

2. Verfahren nach Anspruch 1, dadurch gekennzeichnet, dass der Partialdruck von $NH_3$ mindestens 100 kPa besträgt.

3. Verfahren nach Anspruch 2, dadurch gekennzeichnet, dass der Partialdruck von $NH_3$ zumindest 350 kPa beträgt.

4. Verfahren nach Anspruch 1, dadurch gekennzeichnet, dass das Gasgemisch zumindest zu 90% aus $NH_3$ und $CO_2$ besteht.

5. Verfahren nach Anspruch 1, dadurch gekennzeichnet, dass das Volumenverhältnis zwischen $NH_3$ und $CO_2$ zwischen 1,5—1 und 2,5—1 liegt.

6. Verfahren nach Anspruch 1, dadurch gekennzeichnet, dass der Gesamtdruck im Fliessbett höchstens 2.500 kPa beträgt.

7. Verfahren nach Anspruch 1, dadurch gekennzeichnet, dass der Gesamtdruck im Fliessbett höchstens 1.250 kPa ist.

## Revendications

1. Procédé de préparation de mélamine moyennant la conversion d'urée et/ou de produits de décomposition thermique de celle-ci, dans un lit fluidisé de matériau catalytiquement actif, en présence d'un mélange de gaz contenant du $NH_3$ et du $CO_2$, en utilisant comme gaz de fluidisation un mélange de gaz de $NH_3$ et de $CO_2$, caractérisé en ce que la pression partielle de l'ammoniaque dans le lit fluidisé est de 70 kPa au minimum.

2. Procédé selon la revendication 1, caractérisé en ce que la pression partielle du $NH_3$ est de 100 kPa au minimum.

3. Procédé selon la revendication 2, caractérisé en ce que la pression partielle du $NH_3$ est de 350 kPa au minimum.

4. Procédé selon la revendication 1, caractérisé en ce que le mélange de gaz se compose pour 90% au minimum de $NH_3$ et de $CO_2$.

5. Procédé selon la revendication 1, caractérisé en ce que le rapport en volume entre le $NH_3$ et le $CO_2$ se situe entre 1,5—1 et 2,5—1.

6. Procédé selon la revendication 1, caractérisé en ce que la pression totale dans le lit fluidisé est de 2,500 kPa au maximum.

7. Procédé selon la revendication 1, caractérisé en ce que la pression totale dans le lit fluidisé est de 1,250 kPa au maximum.

FIG.1

0018 696

FIG.2

2